# EUROPÄISCHE PATENTANMELDUNG

(11) **EP 1 602 359 A1**
(43) Veröffentlichungstag der Anmeldung: **07.12.2005**
(21) Anmeldenummer: 05104111.9
(22) Anmeldetag: 17.05.2005
(51) Int. Cl.: A61K 7/48, A61K 7/42, A61K 7/02

(54) **Kosmetische oder dermatologische getränkte Tücher**

(30) Priorität: 02.06.2004 DE 102004027478
(71) Anmelder: Beiersdorf AG, 20253 Hamburg (DE)
(72) Erfinder: Drucks, Anja, 22399, Hamburg (DE); Schlulzig, Mark, 20249, Hamburg (DE); Max, Heiner, 22529, Hamburg (DE); Langhals, Maria, 53229, Bonn (DE)

(57) **Zusammenfassung**

Kosmetische und dermatologische Tücher, wobei die Tücher aus einem einlagigen abrasiven Vlies bestehen, welches mit einer kosmetischen und dermatologischen Tränkungslösung befeuchtet ist, die eine Viskosität von weniger als 2000 mPa·s aufweist, und deren Verwendung.

## Beschreibung

Die vorliegende Erfindung betrifft oberflächenstrukturierte abrasive kosmetische und dermatologische Tücher, welche mit dünnflüssigen kosmetischen und dermatologischen Tränkungslösungen befeuchtet sind. Insbesondere betrifft die Erfindung kosmetische und dermatologische getränkte Pflege- und Reinigungstücher sowie getränkte Tücher zur Bekämpfung von Hautkrankheiten (wie Akne etc.) und zum Abschminken.

Getränkte Tücher finden als Gegenstände des täglichen Bedarfs breiten Einsatz in unterschiedlichsten Bereichen. Sie erlauben unter anderem effiziente und hautschonende Reinigung und Pflege besonders auch in Abwesenheit von (fließendem) Wasser.
Dabei besteht der eigentliche Gebrauchsgegenstand aus zwei Komponenten:
a) einem trockenen Tuch, welches aus Materialien wie Papier und/oder unterschiedlichsten Mischungen aus Natur- oder Kunstfasern aufgebaut ist und
b) einer niederviskosen Tränkungslösung.

Auch oberflächenstrukturierte Tücher sind an sich bekannt. Sie werden auf der Basis von Cellulose hergestellt und finden insbesondere als Haushaltstücher und zur perianalen Reinigung Verwendung. Ihre Struktur wird durch mechanische Prägung mittels Kalanderwalzen erzeugt. Derartige Tücher haben eine geringe Reißfestigkeit bei gleichzeitig großer Rauhigkeit und Härte. Sie eignen sich daher nur bedingt zur Verwendung an der menschlichen Haut.

Aufgabe der vorliegenden Erfindung war es daher, kosmetische oder dermatologische getränkte Tücher zu finden, die die Nachteile des Standes der Technik nicht zeigen und sich insbesondere zur Pflege und/oder Reinigung der Haut eignen.

Es war überraschend und für den Fachmann nicht vorauszusehen, daß
kosmetische und dermatologische Tücher, wobei die Tücher aus einem einlagigen abrasiven Vlies bestehen, welche mit kosmetischen und dermatologischen Tränkungslösungen befeuchtet sind, die eine Viskosität von weniger als 2000 mPa·s aufweisen,
den Nachteilen des Standes der Technik abhelfen.

Die erfindungsgemäßen Tücher stellen die Kombination eines weichen, aber abrasiven Vliesgewebes (nonwoven material) mit dünnflüssigen kosmetischen und dermatologischen Tränkungslösungen dar. Sie sind jeglicher Hinsicht überaus befriedigend und eignen sich dementsprechend ganz besonders, um als Grundlage für Zubereitungsformen mit vielfältigen Anwendungszwecken zu dienen. Die erfindungsgemäßen Tücher zeigen sehr gute sensorische und kosmetische Eigenschaften und zeichnen sich ferner durch hervorragende Hautpflegedaten aus.

Die erfindungsgemäßen Tücher eignen sich sehr gut zur schonenden Reinigung; mit ihrer Hilfe lassen sich Hautschüppchen und Hautunreinheiten sanft entfernen, das Hautbild verfeinern, das Erscheinungsbild von Hautporen verbessern, die Regeneration der Haut fördern, die Haut massieren und beleben sowie die Durchblutung fördern. Dabei weisen sie hervorragende Anwendungseigenschaften wie beispielsweise eine sehr gute Griffigkeit auf.

Gleichzeitig sind die erfindungsgemäßen Tücher dazu geeignet, dermatologisch aktive Wirkstoffe auf die Haut aufzubringen und deren Effizienz durch Verstärkung der dermalen Penetration und durch Durchblutungsförderung in der Haut zu steigern.

Somit eignen sich die erfindungsgemäßen Tücher z. B. zur Behandlung der unreinen, großporigen Haut und der Akne, zur Behandlung der trockenen, schuppigen Haut, der Altershaut, zur Behandlung der Cellulitis, zur aktiven Hautaufhellung ("skin whitening") aber auch zur künstlichen Hautbräunung.

Die kosmetischen und dermatologischen Tränkungslösungen, mit welchen die erfindungsgemäßen Tücher befeuchtet sind, können in verschiedenen Formen vorliegen. Sie sind vorzugsweise dünnflüssig, insbesondere sprühbar und haben z. B. eine Viskosität von weniger als 2.000 mPa·s, bevorzugt weniger als 1.500 mPa·s, insbesondere weniger als 400 mPa·s (Meßgerät: Haake Viskotester VT-02 bei 25 °C). In einer besonders bevorzugten Ausführungsform haben die kosmetischen und dermatologischen Tränkungslösungen im Sinne der vorliegenden Erfindung eine Viskosität von weniger als etwa 200 mPa·s (Meßgerät: Haake Viskotester VT-02 bei 25 °C).

Die Tränkungslösungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Formulierungen können vorzugsweise auch eine Mikroemulsion, eine Pickering-Emulsion, eine sprühbare Emulsion oder eine Hydrodispersion sein.

Darüber hinaus können die erfindungsgemäßen Formulierungen aber auch vorteilhaft in Form von ölfreien Zubereitungen - wie beispielsweise als Gele oder (wäßrige, alkoholische, wäßrig-alkoholische) Lösungen - vorliegen.

Sofern die Tränkungslösung im Sinne der vorliegenden Erfindung eine Lösung oder Dispersion darstellt, können als Lösungsmittel verwendet werden:
■ Wasser oder wäßrige Lösungen
■ Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte, MethylPropandiol, Butylenglycol

Insbesondere werden Gemische der vorstehend genannten Lösungsmittel verwendet. Bei alkoholischen Lösungsmitteln kann Wasser ein weiterer Bestandteil sein.

Sofern die Tränkungslösung eine oder mehrere Wasserphasen enthält, können diese vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, - monoethyl- oder -monobutylether, Diethylenglykolmonomethyl- oder -monoethylether und analoge Produkte sowie Polymere, Schaumstabilisatoren, Elektrolyte, Zuckerderivate und/oder Moisturizer.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Zucker, Aminosäuren, Butylenglycol, Milchsäure, Pyrrolidoncarbonsäure und Harnstoff oder wie z. B. beschrieben in "Dry Skin and Moisturizer-Chemicals & Function" Ed. Marie Loden, Howard I. Maibach, CRC Press, 1999 und wie beschrieben in z. B. "Stratum Corneum Moisturization at the molecular Level, 1994, Dermatology Foundation. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Peptide, Proteine wie z. B. Kollagen, Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Ferner vorteilhaft im Sinne der vorliegenden Erfindung sind wasserfreie Zubereitungen, welche neben einer oder mehreren Ölkomponenten weitere öllösliche Hilfs-, Zusatz- und/oder Wirkstoffe enthalten können.

Sofern die Tränkungslösung eine oder mehrere Ölphasen enthält, werden das oder die Öle im Sinne der vorliegenden Erfindung vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder un gesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner können die Öle vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Vorteilhaft werden die Öle gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wodurch beispielsweise W/S- (Wasserin-Silikon-), S/W- (Silikon-in-Wasser-) Formulierungen und dergleichen mehr entstehen. Es wird allerdings bevorzugt, außer dem Silikonöl oder den Silikonölen einen zusätzlichen Gehalt an anderen Ölphasenkomponenten zu verwenden.

Vorteilhaft werden Cyclomethicon e (z. B. Octamethylcyclotetrasiloxan, Decamethylcyclopentasiloxan, Dodecamethylcyclohexasiloxan) als erfindungsgemäß zu verwendende Silikonöle eingesetzt. Aber auch andere Silikonöle sind vorteilhaft im Sinne der vorliegenden Erfindung zu verwenden, beispielsweise Polydimethylsiloxan oder Poly(methyl-phenylsiloxan).

Besonders vorteilhaft sind ferner Mischungen aus Cyclomethicon und Isotridecylisononanoat, aus Cyclomethicon und 2-Ethylhexylisostearat.

Das oder die Öle werden ferner vorteilhaft aus der Gruppe der Phospholipide gewählt. Die Phospholipide sind Phosphorsäureester acylierter Glycerine. Von größter Bedeutung unter den Phosphatidylcholinen sind beispielsweise die Lecithine, welche sich durch die allgemeine Struktur 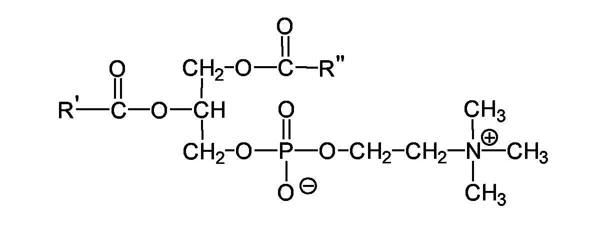 auszeichnen, wobei R' und R" typischerweise unverzweigte aliphatische Reste mit 15 oder 17 Kohlenstoffatomen und bis zu 4 cis-Doppelbindungen darstellen.

Die Tücher im Sinne der vorliegenden Erfindung enthalten vorteilhaft eines oder mehrere waschaktive Tenside der folgenden vier Gruppen A bis D und/oder Tenside wie z. B. beschrieben in WO 99/25318, insbesondere wenn sie als Reinigungstücher verwendet werden sollen:
Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielweise -COO-, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z. B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z. B. Na⁺ |

Typisch für nicht-ionische Tenside sind Polyether-Ketten. Nicht-ionische Tenside bilden in wäßrigem Medium keine Ionen.

### A. Anionische Tenside

Vorteilhaft zu verwendende anionische Tenside sind Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate Carbonsäuren und Derivate, wie

1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-4 Phosphat,

Sulfonsäuren und Salze, wie
1. Acyl-isethionate, z. B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
sowie
Schwefelsäureester, wie
1. Alkylethersulfat, beispielsweise Natrium-, Ammonium-, Magnesium-, MIPA-, TIPA- Laurethsulfat, Natriummyrethsulfat und Natrium C₁₂₋₁₃-Parethsulfat,
2. Alkylsulfate, beispielsweise Natrium-, Ammonium- und TEA-Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkylamidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Die Tränkungslösungen enthalten besonders vorteilhaft eines oder mehrere der vorgenannten waschaktive Tenside. Als "waschaktiv" werden im Sinne der vorliegenden Erfindung Tenside bezeichnet, welche einen HLB-Wert von mehr als 25 haben. Erfindungsgemäß besonders vorteilhafte waschaktive Tenside sind solche, welchen einen HLB-Wert von mehr als 35 haben.

Es ist vorteilhaft im Sinn der vorliegenden Erfindung, wenn der Gehalt an einem oder mehreren waschaktiven Tensiden in der kosmetischen oder dermatologischen Tränkungslösung aus dem Bereich von 5 bis 25 Gew.-%, ganz besonders vorteilhaft von 10 bis 15 Gew.-% gewählt wird, jeweils bezogen auf das Gesamtgewicht der Tränkungslösung.

In einer weiteren vorteilhaften Ausführungsform sind die Tücher im Sinne der vorliegenden Erfindung frei von waschaktiven Tensiden. Ebenso wie Emulsionen von flüssiger und fester Konsistenz als kosmetische Reinigungslotionen bzw. Reinigungscremes Verwendung finden, können auch erfindungsgemäße Tücher, welche frei von waschaktiven Tensiden sind, "Reinigungstücher" darstellen, welche beispielsweise zum Entfernen von Schminken und/oder Make-up oder als milde Reinigungstücher - ggf. auch für unreine Haut - verwendet werden können.

Die Tränkungslösungen für die erfindungsgemäßen kosmetischen und dermatologischen Tücher enthalten weiterhin vorteilhaft auch Konservierungsmittel.

Konservierungsmittel sind antimikrobielle Substanzen, die beim Herstellungsprozeß einem Produkt (Nahrungs- oder Genußmittel, pharmazeutische, kosmetische oder auch chemischtechnische Zubereitungen) in geringen Mengen (gewöhnlich je nach Produkt zwischen ca. 0,0005 % und 1 % Aktivgehalt) zugesetzt werden. Konservierungsmittel sollen Produkte während der Herstellung, der Lagerung und des Gebrauchs vor Verunreinigungen durch Mikroorganismen insbesondere vor den mikrobiell bedingten nachteiligen Veränderungen schützen.

An ein Konservierungsmittel werden grundsätzlich nachfolgende Forderungen gestellt: Es muß ausreichend antimikrobiell wirksam, technologisch anwendbar und gesundheitlich unbedenklich sein. Die gesundheitliche Unbedenklichkeit muß aber auch die fertige Zubereitung, das Handelsprodukt erfüllen. Dabei ist zu berücksichtigen, daß Mikroorganismen in z. B. kosmetischen Mitteln primär produktionsbedingt vorhanden sein oder sekundär durch den Verbraucher in das kosmetische Mittel gelangen können.

Daher muß gewährleistet sein, daß das Fertigprodukt auch über den gesamten Verbrauchszeitraum sicher ist.

Die meisten für eine Konservierung vorgeschlagenen bzw. vorgesehenen Konservierungsmittel wirken bakteriostatisch und fungistatisch, gelegentlich auch bakterizid und fungizid: sie sollen geruch- und geschmacklos und in den zur Anwendung kommenden Dosen nach Möglichkeit löslich, nicht toxisch, hautverträglich und ausreichend wirksam sein. Die Konservierungsmittel müssen, um wirksam zu sein, in dem zu konservierenden Roh- oder Hilfsstoff gelöst sein. Da die meisten Konservierungsmittel besser fett- als wasserlöslich sind, muß damit gerechnet werden, daß z. B. in einer Emulsion, deren wäßrige Phase konserviert werden soll, das in die wäßrige Phase eingearbeitete Konservierungsmittel im Verlauf der Lagerung in die Fettphase auswandert und damit die Konservierung der wäßrigen Phase in Frage gestellt ist. Aus diesem Grunde empfiehlt es sich, eine Kombination von Konservierungsmitteln einzusetzen, d. h. die wäßrige Phase mit einem gut wasserlöslichen Konservierungsmittel, die Fettphase dagegen gleichzeitig mit einem fettlöslichen Konservierungsmittel zu konservieren.

Für ein kosmetisches Präparat braucht zwar in der Regel keine Sterilität gefordert zu werden, es muß aber frei von pathogenen Keimen sein und vor mikrobiell bedingten Veränderungen geschützt werden.

Man sollte berücksichtigen, daß verschiedene Emulsionstypen, wäßrige Lösungen, Suspensionen usw. eine unterschiedliche Konservierung brauchen, daß die konservierende Wirkung einzelner Konservierungsmittel von der Zusammensetzung und den physikalischen Eigenschaften der zu konservierenden Zubereitung abhängig ist, daß mit Interaktionen zwischen dem Konservierungsmittel, den Wirk- und Hilfsstoffen zu rechnen ist, daß verschiedene Wirk- oder Hilfsstoffe Konservierungsmittel adsorbieren und damit möglicherweise inaktivieren können, daß insbesondere in der Zubereitung enthaltene Hydrokolloide konzentrationsabhängig Konservierungsmittel in ihrer antimikrobiellen Aktivität behindern können und daß schließlich - wiederum in Abhängigkeit von der Konzentration und dem Typ des Konservierungsmittels - das Stratum corneum das Konservierungsmittel adsorbiert und das Konservierungsmittel dann möglicherweise zur Permeation und Absorption kommt.

In der Lebensmitteltechnologie zugelassene Konservierungsmittel, welche auch vorteilhaft im Sinne der vorliegenden Erfindung eingesetzt werden können, sind mit ihrer E-Nummer nachfolgend aufgeführt:

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner vorteilhaft sind in der Kosmetik gebräuchliche Konservierungsmittel oder Konservierungshilfsstoffe, wie Dibromdicyanobutan (2-Brom-2-brommethylglutarodinitril), Phenoxyethanol, 3-lod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol vollständig beschrieben im "Handbuch der Konservierungsmittel", ISBN 3818461712.
Weiterhin ist der Einsatz von sogenannten Konservierungsmittelhelfern wie z. B. verschiedenen Polyole n (Methylpropandiol, Propylenglykol, Dipropylenglykol, Butylenglykol, Methylpropandiol, Pentylenglykol, Hexandiol, Caprylyl Glycol, Decylenglykol, Ethylhexylglycerin) vorteilhaft.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid).

Weitere vorteilhaft im Sinne der vorliegenden Erfindung einzusetzende Wirkstoffe sind solche, die den Zustand der Haut positiv beeinflussen, wie insbesondere Wirkstoffe zur positiven Beeinflussung der Altershaut, die die Entstehung von Falten oder auch bestehende Falten vermindern. Vorteilhaft sind insbesondere Biochinone, insbesondere Ubichinon Q10, Carnitin, Biotin, Isoflavon, Cardiolipin, Liponsäure, Anti Freezing Proteine, Hopfen- und Hopfen-Malz-Extrakte. Auch Mittel, die die Restrukturierung des Bindegewebes fördern, wie Isoflavonoide sowie Isoflavonoid-haltige Pflanzenextrakte - wie z. B. Soja- und Klee-Extrakte - können in den erfindungsgemäßen Formulierungen sehr gut verwendet werden. Auch zeigt sich, daß sich die Formulierungen in besonderer Weise eignen, Wirkstoffe zur Unterstützung der Hautfunktionen bei trockener Haut (wie beispielsweise Vitamin C, Biotin, Carnitin, Propionsäure, Grüntee-Extrakte, Eucalyptusöl, Harnstoff und Mineralsalze (wie z. B. NaCl, Meeresmineralien) sowie Osmolyte (wie z. B. Inositol, Betain, quartäre Ammoniumverbindungen)) zu verwenden. In ähnlicher Weise erwies sich die Einarbeitung von Wirkstoffen zur Linderung bzw. positiven Beeinflussung von irritativen Hautzuständen, sei es bei empfindlicher Haut im allgemeinen oder bei durch Noxen gereizter Haut (UV-Licht, Chemikalien), als vorteilhaft. Hier sind Wirkstoffe zu nennen wie Sericoside, verschiedene Extrakte des Süßholzes, Licochalcone, insbesondere Licochalcon A, Silymarin, Silyphos, Dexpanthenol, Inhibitoren des Prostaglandinstoffwechsels, insbesondere der Cyclooxygenase und des Leukotrienstoffwechsels, insbesondere der 5-Lipoxygenase, aber auch des 5-Lipoxygenase Inhibitor Proteins, FLAP. Auch erwies sich die Einarbeitung von Modulatoren der Pigmentierung als vorteilhaft. Hier sind Wirkstoffe zu nennen, die die Pigmentierung der Haut vermindern und so zu einer kosmetisch gewünschten Aufhellung der Haut führen und/oder das Auftreten von Altersflecken reduzieren und/oder bestehende Altersflecken aufhellen. Beispielhaft sei erwähnt Tyrosinsulfat, Dioic acid (8-Hexadecen-1,16-dicarbonsäure) sowie Liponsäure und Liponamid, verschiedene Extrakte des Süßholzes, Kojisäure, Hydrochinon, Arbutin, Alpha-Arbutin, Deoxyarbutin, Fruchtsäuren, insbesondere Alpha-Hydroxy-Säuren (AHAs), Bearberry (Uvae ursi), Ursolsäure, Ascorbinsäure, Grüntee-Extrakte, Aminoguanidin, Pyridoxamin. Besonders bevorzugt sind ferner erfindungsgemäße Formulierungen, die weitere Wirkstoffe enthalten, die eine verstärkte oder schnellere Bräunung der Haut herbeiführen (Advanced Glycation Endproducts (AGE), Lipofuscine, NukleinsäureOligonukleotide, Purine und Pyrimidine, NO-freisetzende Substanzen), sei es mit oder ohne Einfluss von UV-Licht.

Erfindungsgemäße Tränkungslösungen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Herstellung kosmetischer Tücher, deren Anwendung die Haut vor Hautveränderungen schützen sollen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sich derartige Tränkungslösungen zur Herstellung von kosmetischen Tüchern, die vor dem Erscheinungsbild der trockenen bzw. rauhen Haut schützen.

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Bf. Goodrich], beispielsweise Carbopole der Typen 980, 981, 1382, 2984, 5984, ETD 2020, ETD 2050, Ultrez 10, jeweils einzeln oder in Kombination.

Die erfindungsgemäßen Tränkungslösungen können ferner vorteilhaft auch Selbstbräunungssubstanzen enthalten, wie beispielsweise Dihydroxyacteon und/oder Melaninderivate in Konzentrationen von 1 Gew.-% bis zu 8 Gew.-%, bezogen auf das Gesamtgewicht der Tränkungslösung.

Die erfindungsgemäßen kosmetischen und dermatologischen Tücher gemäß der vorliegenden Erfindung können besonders vorteilhaft auch zur Hautaufhellung verwendet werden und dementsprechende Wirkstoffe enthalten. Nicht-limitierende Beispiele sind Vitamin C und seine Derivate, Hydrochinon und seine Derivate (z. B. Arbutin), Niacinamid, Vitamin A und seine Derivate, Alpha-Hydroxysäuren (z. B. Milchsäure), Antioxidanzien und Komplexbildner, Kojisäure, Flavonoide und Isoflavonoide, Dicarbonsäuren wie Azelainsäure oder 8-Hexadecen-1,16-dicarbonsäure, Proteaseinhibitoren (z. B. Tranexaminsäure), Fusarinsäure, Tyrosinderivate, Glabridin, Pflanzenextrakte (z. B. Süßholz-, Tee- oder Maulbeerextrakte wie zitiert in Lee et al. International Journal of Cosmetic Science 19, 291-298, 1997), Plazentaextrakt, Oligo-Peptide (z. B. Agouti-Peptide), Peptidhormonantagonisten (z. B. Antagonisten des Alpha-MSH oder des Endothelins), Membranpotentialblocker (z. B. Dinitrophenol).

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Die erfindungsgemäßen kosmetischen und dermatologischen Tücher können Farbstoffe und/oder Farbpigmente enthalten, insbesondere wenn sie in Form von dekorativen Kosmetika vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus dem Rowe *Colour Index, 3. Auflage, Society of Dyers and Colourists, Bradford, England, 1971* zu wählen.

Ferner vorteilhaft können die erfindungsgemäßen Zubereitungen auch mikrofeine Partikel enthalten:
Es ist vorteilhaft, den mittleren Partikeldurchmesser der verwendeten Partikel zwischen 1 nm und 200 nm, besonders vorteilhaft zwischen 5 nm und 100 nm zu wählen.

Es ist ferner vorteilhaft, die Konzentration aller Partikel größer als 0,1 Gew.-%, besonders vorteilhaft zwischen 0,1 Gew.-% und 30 Gew.-%, bezogen auf das Gesamtgewicht der Zubereitungen, zu wählen.

Vorteilhaft im Sinne der vorliegenden Erfindung sind alle Partikel, die geeignet sind zu mattieren, abzudecken, zu schützen. Im wesentlichen unerheblich für die vorliegende Erfindung ist es, in welcher der gegebenenfalls natürlich vorkommenden Modifikationen die Partikel vorliegen.

Vorzugsweise werden unbehandelte, nahezu reine Pigmentpartikel verwendet, insbesondere solche, welche auch als Farbstoff in der Lebensmittelindustrie und/oder als Absorber von UV-Strahlung in Sonnenschutzmitteln verwendet werden können. Vorteilhaft sind beispielsweise die bei der Firma Merck erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Vorteilhaft sind ferner Pigmente, die oberflächlich wasserabweisend behandelt ("gecoatet") sind. Diese Oberflächenbehandlung kann darin bestehen, daß die Pigmente nach an sich bekannten Verfahren mit einer dünnen hydrophoben Schicht versehen werden.

Besonders vorteilhaft sind TiO₂-Pigmente, beispielsweise die mit Aluminiumstearat beschichteten, unter der Handelsbezeichnung MT 100 T bei der Firma TAYCA erhältlichen.

Eine weitere vorteilhafte Beschichtung der anorganische Pigmente besteht aus Dimethylpolysiloxan (auch: Dimethicon), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaft im Sinne der vorliegenden Erfindung ist ferner die Verwendung einer Mischung verschiedener Pigmenttypen sowohl innerhalb eines Kristalls, beispielsweise als Eisenmischoxid, als auch durch Kombination mehrerer Pigmenttypen innerhalb einer Zubereitung.

Vorzugsweise sind ferner Bornitridpartikel, beispielsweise die im folgenden aufgelisteten Bornitride:
- Handelsname: erhältlich bei
- Boron Nitride Powder: Advanced Ceramics
- Boron Nitride Powder: Sintec Keramik
- Ceram Blanche: Kawasaki
- HCST Boron Nitride: Stark
- Très BN®: Carborundum
- Wacker-Bornitrid BNP: Wacker-Chemie

Vorteilhaft ist es, den mittleren Partikeldurchmesser der verwendeten Bornitridpartikel kleiner als 20 µm, besonders vorteilhaft kleiner als 15 µm zu wählen.

Vorteilhaft sind ferner beispielsweise die bei der Firma Carborundum unter der Handelsbezeichnung Très BN® UHP 1106 erhältlichen, mit Dimethicon behandelten Bornitridpartikel.

Vorteilhaft ist ferner eine Beschichtung der Bornitridpartikel mit Polymethylhydrogensiloxan, einem linearen Polysiloxan, welches auch als Methicone bezeichnet wird. Vorteilhafte, mit Methicone behandelte Bornitridpartikel sind beispielsweise die bei der Firma Carborundum unter der Handelsbezeichnung Très BN® UHP 1107 erhältlichen.

Weitere vorteilhafte Pigmente sind mikrofeine Polymerpartikel.

Vorteilhafte mikrofeine Polymerpartikel sind im Sinne der vorliegenden Erfindung beispielsweise Polycarbonate, Polyether, Polyethylen, Polypropylen, Polyvinylchlorid, Polystyrol, Polyamide, Polyacrylate und dergleichen mehr.

Erfindungsgemäß vorteilhaft sind beispielsweise mikrofeine Polyamid-Partikel, welche unter der Handelsbezeichnung SP-500 bei der Firma TORAY erhältlich sind. Ferner vorteilhaft sind Polyamid 6- (auch: Nylon 6) bzw. Polyamid 12- (auch: Nylon 12) Partikel. Polyamid 6 ist das aus ε-Aminocapronsäure (6-Aminohexansäure) oder ε-Caprolactam aufgebaute Polyamid [Poly(ε-caprolactam)], und Polyamid 12 ist ein Poly(ε-laurinlactam) aus ε-Laurinlactam. Vorteilhaft im Sinne der vorliegenden Erfindung sind beispielsweise Orgasol® 1002 (Polyamid 6) und Orgasol® 2002 (Polyamid 12) von der Firma ELF ATOCHEM.

Weitere vorteilhafte Polymerpartikel sind mikrofeine Polymethacrylate. Solche Partikel sind beispielsweise unter der Handelsbezeichnung POLYTRAP® bei der Firma DOW CHEMICAL erhältlich.

Es ist insbesondere vorteilhaft, wenngleich nicht zwingend, wenn die verwendeten mikrofeinen Polymerpartikel oberflächlich beschichtet sind. Diese Oberflächenbehandlung kann darin bestehen, daß die Pigmente nach an sich bekannten Verfahren mit einer dünnen hydrophilen Schicht versehen werden. Vorteilhafte Beschichtungen bestehen beispielsweise aus TiO₂, ZrO₂ oder auch weiteren Polymeren, wie beispielsweise Polymethylmethacrylat.

Besonders vorteilhafte mikrofeine Polymerpartikel im Sinne der vorliegenden Erfindung sind ferner nach dem in der US-Patentschrift 4,898,913 beschriebenen Verfahren zur hydrophilen Beschichtung hydrophober Polymerpartikel erhältlich.

Vorteilhaft in ist es, den mittleren Partikeldurchmesser der verwendeten mikrofeinen Polymerpartikel kleiner als 100 µm, besonders vorteilhaft kleiner als 50 µm zu wählen. Dabei ist es im wesentlichen unerheblich, in welcher Form (Plättchen, Stäbchen, Kügelchen etc.) die verwendeten Polymerpartikel vorliegen.

Desweiteren ist es vorteilhaft, modifizierte Polysaccharide zu verwenden.

Modifizierte Polysaccharide im Sinne der vorliegenden Erfindung sind beispielsweise durch Umsetzung von Stärke mit mono-, bi- oder polyfunktionellen Reagenzien bzw. OxidationsMitteln in weitgehend polymeranalog verlaufenden Reaktionen erhältlich.

Solche Reaktionen basieren im wesentlichen auf Umwandlungen der Hydroxy-Gruppen der Polyglucane durch Veretherung, Veresterung oder selektive Oxidation. Dabei entstehen z. B. sogenannte Stärkeether und Stärkeester der allgemeinen Strukturformel 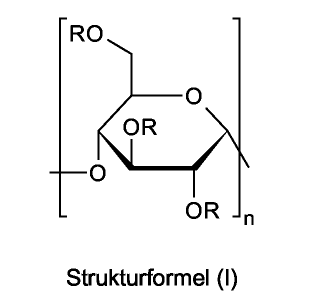 worin R beispielsweise ein Wasserstoff und/oder einen Alkyl- und/oder Aralkylrest (im Fall der Stärkeether) oder ein Wasserstoff und/oder einen organischen und/oder anorganischen Säure-Rest (im Fall der Stärkeester) darstellen kann. Stärkeether und Stärkeester sind vorteilhafte modifizierte Polysaccharide im Sinne der vorliegenden Erfindung.

Besonders vorteilhafte Stärkeether sind z. B. solche, die durch Veretherung von Stärke mit Tetramethylolacetylendiharnstoff erhältlich sind und welche als Amylum non mucilaginosum (nicht quellende Stärke) bezeichnet werden.

Insbesondere vorteilhaft sind ferner Stärkeester und deren Salze, beispielsweise die Natrium- und/oder Aluminiumsalze niedrigsubstituierter Halbester der Stärke, insbesondere Natrium Stärke n-Octenylsuccinat der Strukturformel (I), worin R sich durch die folgende Struktur auszeichnet 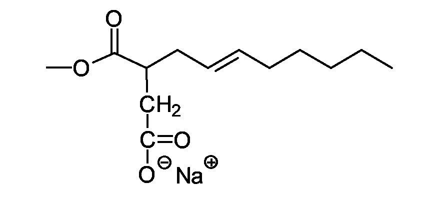 und welches z. B. unter der Handelsbezeichnung Amiogum® 23 bei der Firma CERESTAR erhältlich ist sowie Aluminium Stärke Octenylsuccinate, insbesondere die unter den Handelsbezeichnungen Dry Flo® Elite LL und Dry Flo® PC bei der Firma CERESTAR erhältlichen.

Vorteilhaft ist es, den mittleren Partikeldurchmesser der verwendeten modifizierten Polysaccharide kleiner als 20 µm, besonders vorteilhaft kleiner als 15 µm zu wählen.

Die Liste der genannten modifizierten Polysaccharide soll selbstverständlich nicht limitierend sein. Modifizierte Polysaccharide im Sinne der vorliegenden Erfindung sind auf zahlreichen, an sich bekannten Wegen, sowohl chemischer als auch physikalischer Natur erhältlich.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanz im Sinne der vorliegenden Erfindung ist ferner ist das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches bei 3V Sigma unter der Handelsbezeichnung Uvasorb® K2A erhältlich ist.

Das oder die Benzoxazol-Derivate liegen vorteilhaft in gelöster Form in den erfindungsgemäßen kosmetischen Zubereitungen vor. Es kann ggf. aber auch von Vorteil sein, wenn das oder die Benzoxazol-Derivate in pigmentärer, d. h. ungelöster Form - beispielsweise in Partikelgrößen von 10 nm bis zu 300 nm - vorliegen.

Vorteilhafte UV-Filtersubstanz im Sinne der vorliegenden Erfindung ist ferner der 2-(4'-Diethylamino-2'-hydoxybenzoyl)-benzoesäurehexylester (auch: Aminobenzophenon), welcher sich durch folgende Struktur auszeichnet: 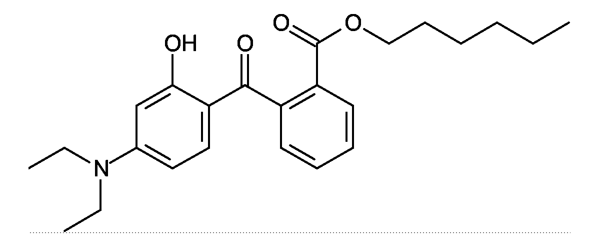 und unter dem Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist;
- Dioctylbutylamidotriazon (INCI: Diethylhexyl Butamido Triazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird;
- 2-[4,6-Bis(2,4-dimethylphenyl)-1,3,5-triazin-2-yl]-5-(octyloxy)phenol (CAS Nr.: 2725-22-6).

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z. B. un ter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein. Vorteilhafte öllösliche Filtersubstanzen sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

Vorteilhafte wasserlösliche Filtersubstanzen sind z. B.:
Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 T erhältlich ist.

Die Liste der genannten UV-Filter, die im Sinne der vorliegenden Erfindung eingesetzt werden können, soll selbstverständlich nicht limitierend sein.

Vorteilhaft im Sinne der vorliegenden Erfindung sind ferner Formulierungen mit einem Gehalt an sogenannten "Peeling-Wirkstoffen", die die natürliche Desquamationsrate -d. h. die Abstoßungsgeschwindigkeit von abgestorbenen, verhornten Schichten - der Haut erhöhen. Hierzu gehören u. a. chemische Peeling-Wirkstoffe wie Alpha-Hydroxysäuren, Saliycylsäure und ihre Derivate, Triflouressigsäure, Benzoylperoxid, Phenol sowie Vitamin A und seine Derivate.

Desweiteren ist der Einsatz von desquamationsfördernden Enzymen vorteilhaft. Bevorzugt sind hydrolytisch wirksame Enzyme (Hydrolasen), die ihre Aktivität auf der Hautoberfläche entfalten können. Hierzu gehören Glycosidasen, Lipasen und insbesondere Proteasen, z. B. Papain, Bromelain, Trypsin, Chymotrypsin, SCCE (stratum corneum chymotrypsin-like enzyme, SCTE (stratum corneum trypsin-like enzyme), Subtilisin und Cathepsine sowie weitere Proteasen, welche z. B. bei Howard Fein in der US-Patentanmeldung US-2003/0026794-A1 zitiert werden.

Weiter vorteilhaft im Sinne der vorliegenden Erfindung sind ferner Formulierungen mit einem Gehalt an sebumregulierenden Wirkstoffen, wie z. B. Carnitin, 8-Hexadecen-1,16-dicarbonsäure, Flavonoide wie Alpha Glucosylrutin, Isoflavonoide wie Genistein und Daidzein, Antiandrogene, 5-alpha-Reduktaseinhibitoren, Retinoide, PPAR-Antagonisten, Inhibitoren der Lipogenese, Biochanin A, Linolsäurederivate, mehrwertige Metallkationen wie z. B. Zink, Kupfer, Mangan, Zirkonium und Aluminium, 2-Indolessigsäure, aber auch Extrakte aus Soja, Coleus, Hopfen, Algen und Kastanie.

Vorteilhaft im Sinne der vorliegenden Erfindung sind ferner Formulierungen mit einem Gehalt an Wirkstoffen mit lokal vasodilatatorischer (durchblutungsfördernder) Wirkung. Hierzu zählen u. a. methylierte Xanthine (Coffein, Thephyllin, Theobromin), sowie Capsaicin, Heparin, Nicotinsäureester, Campher, Menthol, Coumarinderivate, Forskolin, Neuropeptide (z. B. Substance P, Neuropeptid Y) sowie Extrakte aus Pfefferschote, Kletterefeu, Arnika, Rosmarin, Ringelblume, Salbei, Ginseng, Ginkgo biloba, Johanniskraut, Mäusedorn, Geißbart und Orthosiphon sowie Mischungen solcher Wirkstoffe.

Vorteilhaft im Sinne der vorliegenden Erfindung sind ferner Formulierungen mit einem Gehalt an lipolytisch wirksamen Inhaltsstoffen, die zusammen mit der Massagewirkung des Vliesses einen Anti-Cellulitiseffekt fördern. Hierzu gehören z. B. die bereits oben genannten methylierten Xanthine (Coffein, Thephyllin, Theobromin), cAMP, Carnitin und seine Derivate, Cystein, S-Carboxymethylcystein, Thioetherverbindungen sowie Pflanzenextrakte z. B. aus Centella asiatica, Coleus, Yucca, Jasmin, Kaffee, Tee.

Die erfindungsgemäßen Tücher eignen sich ferner hervorragend als Träger für dermatologische Wirkstoffe, z. B. als Träger für gegen Akne wirksame Substanzen. Akne ist eine Hauterkrankung mit verschiedenen Formen und Ursachen, gekennzeichnet durch nicht entzündliche und entzündliche Knötchen, ausgehend von verstopften Haarfollikeln (Komedonen), die zur Pustel-, Abszeß- und Narbenbildung führen kann. Am häufigsten ist die Acne vulgaris, die vorwiegend in der Pubertät auftritt. Ursächliche Bedingungen für die Acne vulgaris sind die Verhornung und Verstopfung der Haarfollikel-Mündung, die vom Blutspiegel der männlichen Sexualhormone abhängige Talgproduktion und die Produktion freier Fettsäuren und gewebeschädigender Enzyme durch Bakterien (*Propionibacterium acnes).*

Daher ist es vorteilhaft, den erfindungsgemäßen Tränkungslösungen gegen Akne wirksame Substanzen zuzugeben, die beispielsweise gegen *Propionibacterium acnes* wirk sam sind (etwa solche, die in DE-OS 42 29 707, DE-OS 43 05 069, DE-OS 43 07 976, DE-OS 43 37 711, DE-OS 43 29 379 beschrieben werden) aber auch andere gegen Akne wirksame Substanzen, beispielsweise all-trans-Retinsäure, 13-cis-Retinsäure und verwandte Stoffe) oder antientzündliche Wirkstoffe, beispielsweise Batylalkohol (α-Octadecylglycerylether), Selachylalkohol (α-9-Octadecenylglycerylether), Chimylalkohol (α-Hexadecylglycerylether) und/oder Bisabolol sowie Antibiotika und/oder Keratolytika.

Keratolytika sind Stoffe, die verhornte Haut (wie z. B. Warzen, Hühneraugen, Schwielen und dergleichen mehr) erweichen, damit sich diese leichter entfernen läßt oder damit sie abfällt bzw. sich auflöst.

Alle gängigen gegen Akne wirksamen Substanzen können vorteilhaft genutzt werden, insbesondere Benzoylperoxid, Vitamin A und seine Derivate (Retinol, Retinsäure, Isotretinoin, Retinylpalmitat), Milchsäure, Glycolsäure, Brenztraubensäure, Macrolide, Tetracycline, Erythromycin, Clindamycin, Bituminosulfonate (Ammonium-, Natrium- und Calcium-Salze von Schieferöl-Sulfonsäuren), Salicylsäure (2-Hydroxybenzoesäure), Hexachlorophen, Miconazol (1-[2-(2,4-Dichlorbenzyloxy)-2-(2,4-dichlorphenyl)-ethyl]-imidazol) und Derivate, Resorcinol, Adapalen (6-[3-(1-Adamantyl)-4-methoxyphenyl]-2-naphthoesäure), Azelainsäure (Nonandisäure), Mesulfen (2,7--Dimethylthianthren, C₁₄H₁₂S₂) sowie Aluminiumoxid, Zinkoxid und/oder feinverteilter Schwefel.

Die Menge der Antiaknemittel (eine oder mehrere Verbindungen) in den Tränkungslösungen beträgt vorzugsweise 0,01 bis 30 Gew.-%, besonders bevorzugt 0,1 bis 20 Gew.-%, insbesondere 1 bis 10 Gew.-%, bezogen auf das Gesamtgewicht der Tränkungslösung.

Erfindungsgemäß vorteilhaft sind Vliese, welche im Rahmen eines Meltblown-Verfahrens erhältlich sind, z. B. in einem Verfahren, bei dem in einem Schritt mit nur einem Extruder für die verwendeten Polymere und mit nur einem Düsenbalken, der nur Spinndüsen einer Größe besitzt, durch Verwendung eines Gemisches von Polymeren unterschiedlicher Eigenschaften ein einlagiges, beidseitig abrasives Vlies hergestellt wird, das ein gleichförmiges Gemisch aus Mikrofasern eines Faserdurchmessers von 1 bis 10 µm und Makrofasern eines Faserdurchmessers von 15 bis 50 µm aufweist.

Erfindungsgemäße einlagige abrasive Vlies mit in weiten Bereichen definierter Abrasivität sowie definiertem Tränkeaufnahme- und Tränkespeicherungsverhalten sind beispielsweise durch Einsatz eines geeigneten Gemisches von Polymeren unterschiedlicher Schmelzindizes erhältlich.

Entsprechendes gilt, wenn Gemische von Polymeren mit unterschiedlicher Molekülstruktur (d. h. linear, verzweigt, vernetzt, statistisches Copolymer, Blockpolymer u. a.), unterschiedlichem Molekulargewicht und/oder Molekulargewichtsverteilung sowie wenn mindestens zwei Polymere, die nicht miteinander mischbar sind, verwendet werden.

Erfindungsgemäß besonders bevorzugt sind einlagige beidseitig abrasive Vliese, bei denen durch Mitverwendung weiterer Polymere und gegebenenfalls von Additiven das Phänomen der nachträglichen Versprödung dicker Meltblown-Fasern weiter verbessert wird und - falls gewünscht - höhere Festigkeits- und Dehnungswerte erreicht werden.

Bei diesen weiteren Polymeren kann es sich beispielsweise handeln um Polyethylen, Polyethylen/Polypropylen-Copolymer oder ein Polypropylen-Blockpolymer mit einem Anteil (üblicherweise 30 % oder weniger, bezogen auf das Polypropylen-Blockpolymer) einer Kautschukphase, die zu gleichen Teilen aus Ethylen und Propylen besteht. Diese Polymere werden den Gemischen gewöhnlich in Mengen von 0,1 bis 20 Gew.-%, vorzugsweise 0,2 bis 10 Gew.- %, insbesondere 0,5 bis 8 Gew.-%, jeweils bezogen auf das Gewicht der gesamten Mischung, zugesetzt.

Die erfindungsgemäßen Vliese können Additive enthalten. Als Additive lassen sich in diesen Fällen grenzflächenaktive Mittel, wie Alkylbenzolsulfonate, Alkansulfonate, Fettalkoholsulfonate, Fettalkoholethersulfate, alpha-Olefinsulfonate, alpha-Estersulfonate, Alkylphosphate, Alkyletherphosphate, Fellalkoholethoxylate, Alkylphenolethoxylate, Fettaminethoxylate, Fettsäureethoxylate, Fettsäureesterethoxylate, Alkoxylate, Alkanolamide, Zuckertenside, Aminoxide, kationische Tenside und amphothere Tenside, insbesondere nichtionische Fluorkohlenstofftenside, und/oder primäre und sekundäre Antioxidantien, wie sterisch gehinderte Phenole, Sulfide, Polysulfide, Dithiocarbamate, Phosphite, Phosphonite, Diphosphonite, und Phosphonate, verwenden. Die Additive werden den Gemischen gewöhnlich in Mengen von 0,01 bis 15 Gew.-%, vorzugsweise 0,01 bis 10 Gew.- %, insbesondere in Mengen von 0,1 bis 5 Gew.-%, jeweils bezogen auf das Gewicht der gesamten Mischung, zugesetzt.

Bei der Herstellung der Vliese können die Additive und/oder Polymere den Gemischen vor Eintritt in den Extruder zugemischt werden oder die einzelnen Bestandteile (d. h. die verschiedenen Polymere) können zusammen mit den Additiven als einzelne Bestandteile in den Extruder eingetragen werden. Die Additive können ferner auch nach dem Spinnprozeß auf die Fasern aufgesprüht werden.

Dem Fachmann auf dem einschlägigen Fachgebiet ist es bekannt, daß durch geeignete Wahl und Einstellung der bestimmenden Produktionsparameter im Meltblown-Verfahren gezielt dicke oder dünne Fasern ausgesponnen werden können.

Wichtige Prozeßparameter in diesem Zusammenhang sind die folgenden: Extrudertemperatur, Temperatur der Schmelze, Temperatur des Düsenbalkens, Durchsatz der Schmelze und Druck, Lufttemperatur, Luftmenge, Design des Düsenbalkens, Abstand des Düsenbalkens von Kollektor, Unterdruck im Kollektor, Geschwindigkeit des Kollektors, Basisgewicht des resultierenden Vlieses und Behandlung mit aerosolartig verteiltem Wasser, das gegebenenfalls Additive enthält.

Um das erfindungsgemäße abrasive Vlies herstellen zu können, ist es darüber hinaus notwendig, dicke und dünne Fasern zusätzlich durch die Wahl geeigneter Polymerparameter, durch die Auswahl geeigneter Polymertypen und gegebenenfalls der Polymermischung sowie gegebenenfalls durch die Verwendung bestimmter Additive gleichzeitig auszuspinnen.

Wichtige Parameter sind in diesem Zusammenhang z. B. der Schmelzindex des Polymers, die Molekülstruktur (d. h. linear, verzweigt, vernetzt, statistisches Copolymer, Blockpolymer u. a.), das Molekulargewicht und/oder die Molekulargewichtsverteilung.

Der sog. Schmelzindex (MFI oder MFR) ist ein Maß für die Schmelzviskosität bei einer festgelegten Temperatur von thermoplastischen Polymeren und gibt die Menge Material an, die in 10 Minuten unter Wirkung einer bestimmten Kraft bei einer festgelegten Temperatur durch eine definierte Düse hindurchläuft. Somit ist der MFI auch ein grober Indikator für das Molekulargewicht und die Molekulargewichtsverteilung. Allerdings weist Bugada in ihrem Aufsatz "Optimizing polymer properties for process and product performance", erschienen in Nonwovens World, August-September 1999, S. 89ff darauf hin, daß bei nahezu gleichen MFIs das Molekulargewicht deutlich variieren kann und umgekehrt.

Die Fasereigenschaften können ferner beispielsweise durch Mischung von Polymeren verschiedener Schmelzindizes zusätzlich individuell gesteuert werden. Um ein erfindungsgemäßes Vlies enthaltend ein Gemisch aus Mikro- und Makrofasern gemäß der obigen Definition gleichzeitig mit einem Extruder und einem Düsenbalken zu produzieren, ist es daher erforderlich, Polymere beispielsweise unterschiedlicher Schmelzindizes, unterschiedlicher Molekülstruktur, unterschiedlichen Molekulargewichts und/oder unterschiedlicher Molekulargewichtsverteilung im Extruder zu schmelzen, ohne daß sich eine resultierende einheitliche Viskosität einstellt. Hierbei muß gewährleistet sein, daß die beiden Viskositäten im wesentlichen ohne Angleichung aneinander bestehen bleiben.

Vorteilhafte Vliese im Sinne der vorliegenden Erfindung sind erhältlich, wenn als Ausgangsmaterialien Polymere verwendet werden, die bereits vorabgebaut sind. Wenn Polymere, die Peroxidverbindungen enthalten, als Bestandteile des erfindungsgemäß zu verwendenden Gemisches verwendet werden, kann in einigen Fällen eine Tendenz zu einer Angleichung der Viskositäten beobachtet werden.

Erfindungsgemäß vorteilhafte Polymere sind z. B. Polyolefine, Polyester, Polyamide, Polyesteramide und Copolymere hiervon, beispielsweise Polyethylen, Polypropylen, Polybutylen, Nylon, Ethylen/Vinylacetat-Copolymere, Ethylen/Propylen-Copolymere oder Polyvinylchlorid. Die genannten Polymere lassen sich entweder als Homopolymer oder als eine Mischung aus unterschiedlichen Polymeren (Copolymere) extrudieren.

So kann es sich bei den zur Herstellung des erfindungsgemäßen abrasiven Vlieses verwendbaren Polymeren beispielsweise um die folgenden handeln:
■ eine Mischung mindestens zweier Homopolymere z. B. aus Polyolefinen wie Polypropylen (PP) oder Polyethylen (PE) oder Gemische zweier Polyethylen- oder Polypropylenhomopolymere unterschiedlicher Schmelzindizes,
■ eine Mischung aus mindestens einem Homopolymer und mindestens einem Copolymer bzw.
■ Terpolymer, z. B. ein Gemisch aus Polypropylen und einem Polypropylen/Polyethylen-Copolymer.
■ eine Mischung aus mindestens zwei Copolymeren bzw. Terpolymeren, z. B. ein Gemisch aus einem Polypropylen/Polyethylen-Copolymer und einem Ethylen/Vinylacetat-Copolymer oder ein Gemisch aus zwei Polypropylen/Polyethylen-Copolymeren unterschiedlicher Schmelzindizes.

Beispiele für erfindungsgemäß bevorzugt eingesetzte Gemische sind ein Gemisch aus Polypropylen eines Schmelzindex von 450 und einem Polypropylen-Blockpolymer eines Schmelzindex von 100, ein Gemisch aus peroxidisch abgebautem Polypropylen eines Schmelzindex von 1400 und einem Polypropylen-Blockpolymer eines Schmelzindex von 100, ein Gemisch aus Polypropylen eines Schmelzindex von 450 und Polypropylen eines Schmelzindex von 1200 und ein Gemisch aus Polypropylen eines Schmelzindex von 450 und Polypropylen eines Schmelzindex von 800.

Gewöhnlich werden erfindungsgemäß Polymere mit einem Schmelzindex (MFR) in einem Bereich zwischen 25 und 1800 g/10 min, vorzugsweise in einem Bereich von 50 bis 1600 g/10 min, insbesondere in einem Bereich von 100 bis 1400 g/10 min, verwendet, wobei die Mischungsverhältnisse der Polymere in weitem Rahmen in einem Bereich von 5-95 % / 95-5 %, vorzugsweise in einem Bereich von 5-80 % / 95-20 %, insbesondere in einem Bereich von 5-40 % / 95-60 %, jeweils bezogen auf ein Gemisch aus Polymer mit niedrigerem Schmelzindex/Polymer mit höherem Schmelzindex variiert werden können.

Die Schmelzindizes besitzen bei den erfindungsgemäß verwendeten Polymeren, wenn der Parameter Schmelzindex ausgewählt ist, um gleichzeitig dicke und dünne Fasern nach dem Meltblown-Verfahren zu spinnen, gewöhnlich einen Unterschied von mindestens 50 Einheiten (g/10 min), vorzugsweise 100 Einheiten und insbesondere 300 Einheiten.

Das massegemittelte Molekulargewicht M_{w} der erfindungsgemäß verwendbaren Polymere liegt vorzugsweise in einem Bereich von 50.000 und 200.000 g/mol, insbesondere in einem Bereich von 70.000 bis 150.000 g/mol.

Für ein abrasives Vlies sind neben Rauhigkeit und Tränkespeichervermögen besonders die physikalischen Eigenschaften wie Bruchkraft und Bruchdehnung wichtig. Durch eine geeignete Auswahl der Polymermischung, z. B. durch Verwendung eines Polyethylen/Polypropylen-Copolymers, insbesondere eines solchen mit einem Polyethlyengehalt von nicht mehr als 20 %, wobei das Ethylen in einer Kautschukphase vorliegt, können diese Eigenschaften des Polypropylens stark verbessert werden.

Weitgehend bekannt und für solche Fälle einsetzbar sind Mischungen von Polypropylen mit seinen verwandten Polymeren aus der Familie der Polyolefine und hier besonders mit Polyethylen. Noel und Carley beschreiben in ihrem Aufsatz "Properties of Polypropylene-Polyethylene-Blends", Polymer Engeneering And Science, February 1975, Vol. 15, No. 2, S. 117ff die Bruchkraft-erhöhende Zugabe von bis zu 10 % Polyethylen zu einem Polypropylen. Jedoch werden solche Fasern typischerweise sehr weich, was dem gewünschten Abrasiveffekt des Vlieses entgegenläuft. Derartige weiche Fasern treten auch bei der Verwendung von Polypropylen/Polyethylen-Copolymeren auf, die aus Ethylen und Propylen polymerisiert werden (vgl. z. B. PCT/WO 98/39384).

Erfindungsgemäß vorteilhaft sind daher Vliese, welche durch Mitverwendung eines Copolymers, bei dem das Polyethylen lediglich in einer Kautschukphase vorliegt, erhältlich sind, wobei diese Vliese bei gleichzeitiger hervorragender Abrasivität nicht spröde sind und ihre Bruchdehnung sehr stark erhöht wird (größer 20 %). Ein in diesem Zusammenhang verwendbares Copolymer ist beispielsweise ein handelsübliches Polypropylen-Blockpolymer mit einer geringen Kautschukphase, die aus je ca. 50 % Ethylen und Propylen besteht.

Die Faserdicke der Mikrofasern liegt erfindungsgemäß in einem Bereich von 1 bis 10 µm, vorzugsweise in einem Bereich von 2 bis 8 µm, und insbesondere in einem Bereich von 3 bis 7 µm. Die Faserdicke der Makrofasern liegt erfindungsgemäß in einem Bereich von 15 bis 80 µm, vorzugsweise in einem Bereich von 20 bis 60 µm, und insbesondere in einem Bereich von 30 bis 50 µm.

Es ist vorteilhaft im Sinne der vorliegenden Erfindung, wenn der zahlenmäßige Anteil der Makrofasern in dem erfindungsgemäßen Vlies - bezogen auf eine Flächeneinheit des Vlieses - kleiner als 50 %, insbesondere kleiner als 40 % ist.

Beim Ausspinnen des Polymergemisches kann ferner ein Teil der gebildeten klebrigen Mikro- bzw. Makrofasern auf dem Kollektor bei der Vliesbildung mit benachbarten Fasern zusammenkleben, so daß neben den Mikro- und Makrofasern auch dickere Faserbündel im erfindungsgemäßen Vlies vorliegen können. Die Dicke dieser Faserbündel kann in solchen Fällen gewöhnlich zwischen 50 und 300 µm, vorzugsweise zwischen 70 und 200 µm, insbesondere zwischen 80 und 150 µm liegen.

Es ist vorteilhaft im Sinne der vorliegenden Erfindung, wenn ein mengenmäßiger Anteil von mehr als 10 % der gesamten Fasern in Form von Faserbündeln vorliegt, beispielsweise um die abrasiven Eigenschaften des erfindungsgemäßen Vlieses zu verbessern.

Derartige Vliese sind beispielsweise erhältlich, wenn ein Gemisch aus einem Polypropylen mit einem höheren Schmelzindex und einem Copolymer mit einem geringeren Schmelzindex, der z. B. kleiner als 25 % des Schmelzindex des verwendeten Polypropylens ist, verwendet wird.

Bei Verwendung von Polymergemischen mit stark unterschiedlichen Schmelzindizes, die beide üblicherweise kein oder nur sehr wenig Restperoxid enthalten, kommt es nicht zur Angleichung der Viskositäten, sondern zu zwei unterschiedlich viskosen, gering vermischten Schmelzen, so daß die Bildung von Faserbündeln in geringerem Umfang auftritt und statistisch verteilt über den gesamten Düsenbalken stattfindet. Dicke Faserbündel sowie Makro- und Mikrofasern verteilen sich somit ungeordnet in x-, y- und z-Richtung im Vlies.

Den erfindungsgemäßen Polymermischungen können ferner Additive zugegeben werden, die einen Beitrag leisten können, um spezielle Vlieseigenschaften zu liefern oder zu erhalten. Beispiele für erfindungsgemäß verwendbare Additive sind die folgenden:
■ Hydrophilierungsmittel, beispielsweise die oben genannten nichtionischen Tenside, insbesondere Sulfobernsteinsäureesterderivate oder fluorchemische Tenside;
■ Stabilisatoren gegen oxidative Degradation (z. B. durch Restperoxid, (Luft-) Sauerstoff, (Sonnen-) Licht, Wasser etc.), wie Amine, Phenole, Phosphite, Phosphonite, Diphosphonite oder Thioester und/oder
■ Farbstoffe zur Färbung der Fasern, wie sie besipielsweise von der Fa. Clariant unter der Bezeichnung Sanylene TM vertrieben werden.

Diese Additive können den erfindungsgemäß verwendeten Polymermischungen in Mengen von 0,01 bis 15 Gew.-%, vorzugsweise in Mengen von 0,01 bis 10 Gew.-%, insbesondere von 0,1 bis 5 Gew.-%, jeweils bezogen auf das Gewicht des Polymergemisches, zugesetzt werden.

Die erfindungsgemäß hergestellten einlagigen abrasiven Vliese lassen sich durch die folgenden Parameter beschreiben:
■ Flächengewicht (EDANA-Norm 403 89),
■ Bruchkraft und Bruchdehnung (EDANA-Norm 20289),
■ möglichst niedrige Versprödungsneigung,
■ Abrasivität,
■ Widerstand gegen Fusseln und Abrasion,
■ Tränkehaltevermögen,
■ Tränkeaufnahme,
■ Faserdurchmesser,
■ Porengröße und Porengrößenverteilung,
■ Kontaktwinkel.

Das erfindungsgemäße abrasive Vlies besitzt gewöhnlich ein Flächengewicht von 15 bis 150 g/m, vorzugsweise ein Flächengewicht von 25 bis 80 g/m.

Die erfindungsgemäßen abrasiven Vliese weisen vorteilhaft eine endgültige Bruchkraft von in Maschinenrichtung (MD) mindestens 10 N/50 mm, vorzugsweise mindestens 15 N/50 mm, und quer zur Maschinenrichtung (CD) gewöhnlich mindestens bei 5 N/50 mm, vorzugsweise bei 10 N/50 mm liegt.

Die Bruchdehnung des erfindungsgemäßen abrasiven Vlieses liegt gewöhnlich über 10 %, vorzugsweise über 20 % sowohl in MD als auch in CD. Die Bruchdehnung verbessert sich, wenn das Vlies mit der Tränkungslösung befeuchtet ist.

Das Tränkehaltevermögen sowie die Tränkeaufnahme sind stark vom Faserdurchmesser, der daraus resultierenden Porengrößenverteilung, der Vliesdichte und den oberflächenchemischen Eigenschaften des Vlieses determiniert. Zur Ermittlung der Tränkeaufnahme wird gewöhnlich eine definierte Fläche trocken ausgewogen, dann mit einer Versuchstränke (Tränkungslösung) getränkt, leicht ausgedrückt und naß gewogen. Das Gewicht der aufgenommenen Tränke, das sich aus der Differenz der beiden Meßwerte errechnet, wird in Relation zum Flächengewicht gesetzt und gibt die Tränkeaufnahme in Prozent an. Die Tränkeaufnahme des erfindungsgemäßen abrasiven Vlieses liegt gewöhnlich zwischen 100 und 700 Trockengewichtsprozent, bevorzugt zwischen 100 und 500 Trockengewichtsprozent.

Der Faserdurchmesser der im erfindungsgemäßen abrasiven Vlies vorkommenden Fasern kann durch rasterelektronenmikroskopische (REM) Untersuchung bestimmt werden.

Die Porengröße läßt sich mittels eines Porosimerters bestimmen, welches Quecksilber mit zunehmendem Druck in die Vliesporen preßt. Aus der Druckkurve kann die Anzahl der Poren, Porengrößenverteilung sowie das gesamte Porenvolumen abgeleitet werden. Eine gute Tränkespeicherung zeigen erfindungsgemäße Vliese mit einem Porenvolumenanteil an Poren, die kleiner als 6 µm im Durchmesser sind, von mehr als 50 %, vorzugsweise mehr als 70%

Das Tränkehaltevermögen läßt sich z. B. durch Lagertests bestätigen. Dabei werden mehrere Stapel von 30 Einzelblättern eines erfindungsgemäßen abrasiven Vlieses mit einer bestimmten Menge einer definierten Tränke befeuchtet. Dann wird von den Blättern eines Stapels die aufgenommene Tränkemenge bestimmt, die zu einem Tränkeprofil innerhalb des Stapels führt. Ferner werden weitere Stapel desgleichen erfindungsgemäßen Vlieses in einer dichten Verpackung einen Monat bei Raumtemperatur und bei 40 °C gelagert. Anschließend wird ebenfalls wie beim ersten Stapel das Tränkeprofil in den Stapeln gemessen.

Das erfindungsgemäße abrasive Vlies kann auch über längere Zeit in einer Packung gleichmäßig feucht im gesamten Stapel gelagert werden, ohne daß die oben liegenden Tücher austrocknen, während sich am Boden der Packung aus den Tüchern herausgelaufene Tränke ansammelt.

Das erfindungsgemäße Vlies läßt sich wie oben ausgeführt nach dem Meltblown-Verfahren produktionsmäßig in einem Schritt mit nur einem Extruder für die verwendeten Polymere und mit nur einem Düsenbalken, der nur Spinndüsen einer Größe besitzt, durch Verwendung eines Gemisches von Polymeren unterschiedlicher Eigenschaften herstellen. Hierbei können die verwendeten Polymere sowie gegebenenfalls mitverwendete Additive in dem gewünschten Mischungsverhältnis aus einem Dosiergerät in den Extruder eingetragen werden. Dort wird das Gemisch nach einem geeigneten Temperaturprofil (dieses ist dem Fachmann auf dem einschlägigen Fachgebiet bekannt oder läßt sich durch Routineversuche leicht bestimmen) und verläßt den Extruder üblicherweise mit einer Temperatur zwischen 180 und 300 °C. Eine Pumpe befördert die Schmelze mit einem Durchsatz, der üblicherweise mindestens 50 kg/h beträgt, zum Düsenbalken. Der Abstand zwischen Kollektor und Düsenbalken liegt hierbei gewöhnlich zwischen 25 und 50 cm. Die Geschwindigkeit des Kollektors richtet sich nach dem gewünschten Flächengewicht des Vlieses bei gegebenem Durchsatz der Schmelze und gegebener Breite des verwendeten Düsenbalkens. Die Temperatur der üblicherweise verwendeten heißen Druckluft liegt gewöhnlich zwischen 270 und 320 °C, je nach dem verwendeten Polymergemisch sowie gegebenenfalls zugesetzten Additiven. Der Luftdurchsatz variiert ebenso wie die Lufttemperatur in Abhängigkeit von dem verwendeten Polymergemisch sowie gegebenenfalls zugesetzten Additiven zwischen 250 und 500 m/h.

Darüber hinaus können die Fasern auch eingefärbt sein, um die optische Attraktivität des Vlieses betonen und/oder erhöhen zu können. Die Fasern können zusätzlich UV-Stabilsatoren und/oder Konservierungsmittel enthalten.

Die nachfolgenden Beispiele sollen die erfindungsgemäßen Tränkungslösungen verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

### Beispiel 1

| Bestandteil | Menge / Gew.-% |
|---|---|
| Paraffinum Liquidum | 99,8 |
| Parfum | 0,2 |

### Beispiel 2: Mikroemulsion

| Bestandteil | Menge / Gew.-% |
|---|---|
| Wasser | 82,0 |
| Paraffinum Liquidum | 8,0 |
| Glycerin | 5,0 |
| Octylstearat | 2,0 |
| Glyceryl Stearate, Ceteareth-20, Ceteareth-12, Cetearyl Alcohol, Cetyl Palmitate | 1,5 |
| Phenoxyeheanol, Methylparaben, Ethyl-paraben, Propylparaben, Butylparaben, Isobutylparaben | 0,5 |
| Parfum | 0,4 |
| Ceteareth-20 | 0,3 |
| Methylparaben | 0,3 |
| Summe: | 100,0 |

### Beispiel 3: Mikroemulsion

| Bestandteil | Menge / Gew.-% |
|---|---|
| Wasser | 75,0 |
| Paraffinum Liquidum | 0,5 |
| Glycerin | 7,0 |
| Octylstearat | 1,0 |
| Glyceryl Stearate, Ceteareth-20, Cetea-reth-12, Cetearyl Alcohol, Cetyl Palmitate | 3,0 |
| Phenoxyeheanol, Methylparaben, Ethyl-paraben, Propylparaben, Butylparaben, Isobutylparaben | 0,5 |
| Parfum | 2,0 |
| Ceteareth-20 | 10,0 |
| Methylparaben | 1,0 |
| Summe: | 100,0 |

### Beispiel 4: wäßrige Tränkungslösung

| Bestandteil | Menge / Gew.-% |
|---|---|
| Wasser | 96,89 |
| Butylenglykol | 1,0 |
| PEG-40 Hydrogenated Castor Oil | 0,8 |
| Phenoxyehanol, Methylparaben, Ethyl-paraben, Propylparaben, Butylparaben, Isobutylparaben | 0,65 |
| Kaliumsorbat | 0,3 |
| Parfum | 0,2 |
| Zitronensäure | 0,16 |
| Summe: | 100,0 |

### Beispiel 5: wäßrige Tränkungslösung

| Bestandteil | Menge / Gew.-% |
|---|---|
| Wasser | 95,0 |
| Butylenglykol | 1,0 |
| PEG-40 Hydrogenated Castor Oil | 1,0 |
| Phenoxyethanol,Methylparaben, Ethyl-paraben, Propylparaben, Butylparaben, Isobutylparaben | 1,5 |
| Kaliumsorbat | 0,5 |
| Parfum | 0,5 |
| Zitronensäure | 0,5 |
| Summe: | 100,0 |

### Beispiel 6

| Bestandteil | Menge / Gew.-% |
|---|---|
| Cyclomethicone | 65,5 |
| Dimethicone | 20,0 |
| Silikongum | 7,0 |
| Phenyltrimethylmethicone | 7,0 |
| Parfum | 0,5 |
| Summe: | 100,0 |

### Beispiel 7: alkoholische Tränkungslösung

| Bestandteil | Menge / Gew.-% |
|---|---|
| Ethanol | 60,0 |
| Wasser | 34,5 |
| Glycerin | 5,0 |
| Parfum | 0,5 |
| Summe: | 100,0 |

### Beispiel 8: alkoholische Tränkungslösung

| Bestandteil | Menge / Gew.-% |
|---|---|
| Ethanol | 60,0 |
| Wasser | 24,0 |
| Glycerin | 5,0 |
| Isopropylalkohol | 5,0 |
| Ethylenediamine | 1,0 |
| Dexpanthenol | 1,0 |
| Carbomer | 3,0 |
| Parfum | 0,5 |
| Farbstoff | 0,5 |
| Summe: | 100,0 |

### Beispiel 9: After Sun-/Hautpflege-Mikroemulsion

| Bestandteil | Menge / Gew.-% |
|---|---|
| Ceteth-15 | 6 |
| Glycerylisostearate | 2 |
| Cetyl Alkohol | 1 |
| Dicaprylyl Carbonate | 5 |
| Octyldodecanol | 3 |
| Cylomethicone | 1 |
| Butylene Glycol | 3 |
| Ethanol | 5 |
| DMDM Hydantoin | 0,6 |
| Octoxyglycerin | 1 |
| Antioxydantien | 0,5 |
| Parfuem, | 0,5 |
| Farbstoffe | 0,3 |
| Wasser | ad 100 |

### Beispiel 10: nicht fettende Körperpflegeemulsion

| Bestandteil | Gew.-% |
|---|---|
| Ceteareth-12 | 6 |
| Glyceryl Stearate | 3,5 |
| Cetyl Palmitate | 3 |
| Dicaprylyl Ether | 5 |
| Cycolmethicone | 3 |
| Phenyl Trimethicone | 1 |
| Paraffinwax | 2 |
| Glycerin | 7,5 |
| Parabene | 1 |
| Phenoxyethanol | 1 |
| AGR | 0,5 |
| Parfuem | 0,5 |
| Farbstoffe | 0,5 |
| Wasser | ad 100 |

### Beispiel 11: Sonnenschutzmittel für seidiges Hautgefühl

| Bestandteil | Gew.-% |
|---|---|
| Ceteareth-20 | 5,5 |
| Glyceryl Stearate | 4 |
| Stearyl Alkohol | 3 |
| Dicaprylyl Ether | 5 |
| Octyldodecanol | 3 |
| Phenyl Trimethicone | 1 |
| Bis-Ethylhexyloxyphenol Methoxyphenyl Triazine | 2 |
| Octocrylene | 7 |
| Diethylhexyl Butamido Triazone | 1 |
| Ethylhexyl Methoxycinnamate | 4 |
| Butylene Glycol | 1 |
| Vitamin E Acetat | 1 |
| PVP/Hexadecene Copolymer | 1 |
| Parabene | 1 |
| Antioxydantien | 0,5 |
| Parfuem | 0,5 |
| Wasser | ad 100 |

### Beispiel 12: Sonnenschutzformulierung

| Bestandteil | Gew.-% |
|---|---|
| Ceteareth-20 | 6,5 |
| Glyceryl Stearate | 2 |
| Stearyl Alkohol | 1 |
| Dicaprylyl Carbonate | 5 |
| Octyldodecanol | 3 |
| C12-15 Alkyl Benzoate | 1 |
| Titandioxid | 2 |
| Bis-Ethylhexyloxyphenol Methoxyphenyl Triazine | 2 |
| Octocrylene | 7 |
| Ethylhexyl Methoxycinnamate | 4 |
| Parabene | 1 |
| Antioxydantien | 0,5 |
| Parfuem | 0,5 |
| Wasser | ad 100 |

### Beispiel 13: Sonnenschutzformulierung

| Bestandteil | Gew.-% |
|---|---|
| Steareth-20 | 6,5 |
| Glycerylisostearate | 2 |
| Cetyl Alkohol | 1 |
| Dicaprylyl Carbonate | 5 |
| Shea Butter | 3 |
| C12-15 Alkyl Benzoate | 1 |
| Bis-Ethylhexyloxyphenol Methoxyphenyl Triazine | 2 |
| Butylmethoxydibenzoylmethane | 1 |
| Ethylhexyl Triazone | 2 |
| Phenylbenzimidazol Sulfonsäure | 2 |
| Ethylhexyl Methoxycinnamate | 4 |
| Glycerin | 10 |
| Tricontanyl PVP | 1 |
| Citrat-Puffer | 1 |
| Parabene | 1 |
| Antioxydantien | 0,5 |
| Parfuem | 0,5 |
| Wasser | ad 100 |

### Beispiel 14: Sonnenschutzformulierung

| Bestandteil | Gew.-% |
|---|---|
| Ceteareth-30 | 7 |
| Glycerylisostearate | 2,5 |
| Cetyl Alkohol | 1 |
| Dicaprylyl Carbonate | 4 |
| Capric/Caprylic Triglyceride | 2 |
| C12-15 Alkyl Benzoate | 6 |
| Methylen Bis-Benzotriazolyl Tetrame-thylbutylphenol | 2 |
| Butyl Methoxydibenzoylmethane | 2 |
| Ethylhexyl Triazone | 4 |
| Bis-Imidazylat | 2 |
| Methylbenzylidene Camphor | 4 |
| Glycerin | 5 |
| PVP Hexadecene Copolymer | 1 |
| Parabene | 1 |
| Antioxydantien | 0,5 |
| Parfuem | 0,5 |
| Wasser | ad 100 |

### Beispiel 15: Sonnenschutzformulierung

| Bestandteil | Gew.-% |
|---|---|
| Ceteareth-20 | 7,5 |
| Glyceryl Stearate | 3 |
| Cetyl Palmitate | 1,5 |
| Dicaprylyl Carbonate | 5 |
| Cocoglycerides | 2 |
| C12-15 Alkyl Benzoate | 6 |
| Barium Sulfate | 2 |
| Bis-Ethylhexyloxyphenol Methoxyphenyl Triazine | 2 |
| Ethylhexyl Triazone | 4 |
| Bis-Imidazylat | 1 |
| Phenylbenzimidazol Sulfonsäure | 2 |
| Methylbenzylidene Camphor | 4 |
| PVP Hexadecene Copolymer | 1 |
| NaOH | 0,5 |
| Parabene | 1 |
| Antioxydantien | 0,5 |
| Parfuem | 0,5 |
| Wasser | ad 100 |

### Beispiel 16: After Sun-/Hautpflege-Formulierung

| Bestandteil | Gew.-% |
|---|---|
| Ceteth-15 | 6 |
| Glycerylisostearate | 2 |
| Cetyl Alkohol | 1 |
| Dicaprylyl Carbonate | 5 |
| Shea Butter | 1 |
| Octyldodecanol | 3 |
| Cylomethicone | 1 |
| Mineral Oil | 2 |
| Ethanol | 5 |
| Parabene | 1 |
| Antioxydantien | 0,5 |
| Parfuem | 0,5 |
| Wasser | ad 100 |

### Öl - Beispiele

### Öl-1

| Bestandteil | Gew.-% |
|---|---|
| Capric/Caprylic Triglyceride | 2 |
| C12-15 Alkyl Benzoate | 6 |
| Butyl Methoxydibenzoylmethane | 2 |
| Ethylhexyl Triazone | 2 |
| Bis-Ethylhexyloxyphenol Methoxyphenyl Triazine | 1 |
| Methylbenzylidene Camphor | 4 |
| Shea Butter | 1 |
| Butylene Glycol Dicaprate/Dicaprylate | 3 |
| Dimethicone | 5 |
| Parabene | 1 |
| Antioxydantien | 0,5 |
| Parfuem | 0,5 |
| Mineraloil | ad 100 |

### Öl-2

| Bestandteil | Gew.-% |
|---|---|
| Dicaprylyl Carbonate | 5 |
| Bis-Ethylhexyloxyphenol Methoxyphenyl Triazine | 2 |
| Ethylhexyl Triazone | 4 |
| Methylbenzylidene Camphor | 4 |
| Shea Butter | 1 |
| Octyldodecanol | 3 |
| Cylomethicone | 1 |
| Vitamin E | 1 |
| Parfuem | 0,5 |
| Mineraloil | ad 100 |

### Öl-3

| Bestandteil | Gew.-% |
|---|---|
| Dicaprylyl Carbonate | 5 |
| Bis-Ethylhexyloxyphenol Methoxyphenyl Triazine | 2 |
| Diethylhexyl Butamido Triazone | 4 |
| Methylbenzylidene Camphor | 1 |
| Shea Butter | 1 |
| Phenyltrimethicone | 1 |
| Vitamin E | 2 |
| Parfuem | 0,5 |
| Cyclomethicone | ad 100 |

### Öl-4

| Bestandteil | Gew.-% |
|---|---|
| Ethylhexyl Methoxycinnamate | 10 |
| Dicaprylyl Carbonate | 5 |
| Bis-Ethylhexyloxyphenol Methoxyphenyl Triazine | 2 |
| Diethylhexyl Butamido Triazone | 4 |
| Octocrylene | 5 |
| Shea Butter | 1 |
| Phenyltrimethicone | 1 |
| Vitamin E | 1 |
| Parfuem | 1 |
| Cyclomethicone | ad 100 |

### Wäßrige Formulierung:

| Bestandteil | Gew.-% |
|---|---|
| Bis-Imidazylat | 1 |
| Phenylbenzimidazol Sulfonsäure | 2 |
| Glycerin | 10 |
| Parabene | 1 |
| Antioxydantien | 0,5 |
| Parfuem | 0,5 |
| Wasser | ad 100 |

## Patentansprüche

1. Kosmetisches oder derma tologisches T uch, wobei das Tuch aus einem einlagigen abrasiven Vlies besteht, welches mit einer kosmetischen und/oder dermatologischen Tränkungslösung befeuchtet ist, die eine Viskosität von weniger als 2000 mPa·s aufweist.

2. Tuch nach Anspruch 1, **dadurch gekennzeichnet, daß** das Gewichtsverhältnis des ungetränkten Tuchs zu der Tränkungslösung aus dem Bereich von 1 : 1 bis 1 : 5 gewählt wird.

3. Tuch nach einem der Ansprüche 1 oder 2, **dadurch gekennzeichnet, daß** die Viskosität der Tränkungslösung kleiner als 400 mPa·s gewählt wird.

4. Tuch nach einem der vorhergehenden Ansprüche, **dadurch gekennzeichnet, daß** die Tränkungslösung eine oder mehrere Ölkomponenten enthält.

5. Tuch nach einem der vorhergehenden Ansprüche, **dadurch gekennzeichnet, daß** die Tränkungslösung einen oder mehrere Wirkstoffe enthält.

6. Tuch nach Anspruch 5, **dadurch gekennzeichnet, daß** der oder die Wirkstoffe aus der Gruppe der natürlichen Wirkstoffe und/oder deren Derivate gewählt wird.

7. Tuch nach Anspruch 6, **dadurch gekennzeichnet, daß** der oder die Wirkstoffe aus der Gruppe: alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin sowie 8-Hexadecen-1,16-dicarbonsäure gewählt wird.

8. Tuch nach einem der vorhergehenden Ansprüche, **dadurch gekennzeichnet, daß** die Tränkungslösung eine oder mehrere Selbstbräunungssubstanzen enthält.

9. Tuch nach einem der vorhergehenden Ansprüche, **dadurch gekennzeichnet, daß** die Tränkungslösung ein oder mehrere waschaktive Tenside mit einem HLB-Wert von mehr als 25 enthält.

10. Tuch nach einem der vorhergehenden Ansprüche, **dadurch gekennzeichnet, daß** die Tränkungslösung eine oder mehrere durchblutungsfördernde Substanzen enthält.

11. Tuch nach einem der vorhergehenden Ansprüche, **dadurch gekennzeichnet, daß** die Tränkungslösung einen oder mehrere Antiaknewirkstoffe enthält.

12. Tuch nach einem der vorhergehenden Ansprüche, **dadurch gekennzeichnet, daß** die Tränkungslösung einen oder mehrere Peeling-Wirkstoffe, die die natürliche Desquamationsrate der Haut erhöhen, enthält.

13. Tuch nach einem der vorhergehenden Ansprüche, **dadurch gekennzeichnet, daß** das Vlies beidseitig abrasiv ist.

14. Tuch nach einem der vorhergehenden Ansprüche, **dadurch gekennzeichnet, daß** das Vlies ein gleichförmiges Gemisch aus Mikrofasern eines Faserdurchmessers von 1 bis 10 µm und Makrofasern eines Faserdurchmessers von 15 bis 50 µm aufweist.

15. Tuch nach einem der vorhergehenden Ansprüche, **dadurch gekennzeichnet, daß** das Vlies eine Mischung mindestens zweier Homopolymere unterschiedlicher Schmelzindizes enthält.

16. Tuch nach einem der vorhergehenden Ansprüche, **dadurch gekennzeichnet, daß** das Vlies eine Mischung aus mindestens einem Homopolymer und mindestens einem Copolymer enthält.

17. Tuch nach einem der vorhergehenden Ansprüche, **dadurch gekennzeichnet, daß** das Vlies ein Terpolymer, z. B. ein Gemisch aus Polypropylen und einem Polypropylen/Polyethylen-Copolymer, enthält.

18. Tuch nach einem der vorhergehenden Ansprüche, **dadurch gekennzeichnet, daß** das Vlies eine Mischung aus mindestens zwei Copolymeren bzw. Terpolymeren unterschiedlicher Schmelzindizes enthält.

19. Verwendung von Tüchern nach einem der Ansprüche zur Reinigung und/oder Pflege der Gesichtshaut.

20. Verwendung von Tüchern nach einem der Ansprüche als Abschminktuch.

21. Verwendung von Tüchern nach einem der Ansprüche als Peelingtuch.
